(19) Europäisches Patentamt / European Patent Office / Office européen des brevets

(11) EP 3 081 561 A1

(12) EUROPEAN PATENT APPLICATION

(43) Date of publication:
**19.10.2016 Bulletin 2016/42**

(21) Application number: **15163318.7**

(22) Date of filing: **13.04.2015**

(51) Int Cl.:
***C07D 311/12*** *(2006.01)*
***C07F 9/54*** *(2006.01)*
***C07D 409/04*** *(2006.01)*
***G01N 33/00*** *(2006.01)*
***G01N 33/18*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicant: **Ernst-Moritz-Arndt-Universität Greifswald**
**17487 Greifswald (DE)**

(72) Inventors:
- **Ghosh, Ashta Chandra**
  **17489 Greifswald (DE)**
- **Schulzke, Carola**
  **17493 Greifswald (DE)**

(74) Representative: **Wablat Lange Karthaus Anwaltssozietät**
**Potsdamer Chaussee 48**
**14129 Berlin (DE)**

(54) **MERCURY QUICK TEST**

(57) The present invention is related Nickel complexes according to formula I,

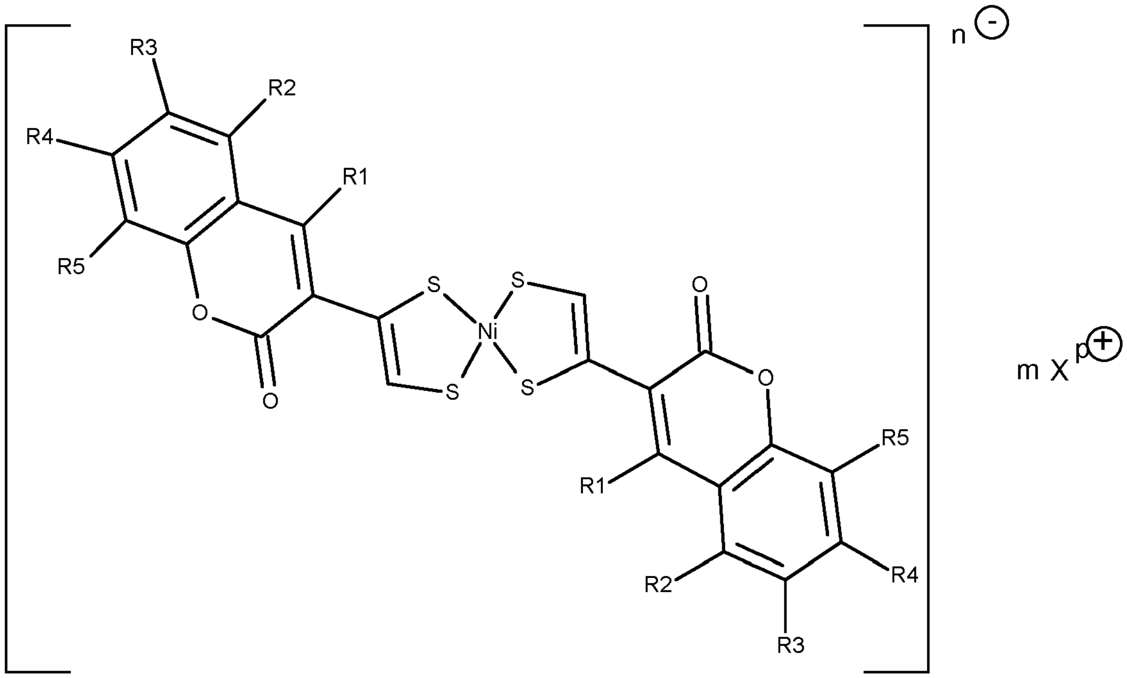

I

wherein
Nickel has the oxidation state II or III,
n, m are each 1 or 2,
p is 1 or 2,



Printed by Jouve, 75001 PARIS (FR)

(Cont. next page)

each group R1-R5 is independently selected from hydrogen, C1-C5-alkyl, C1-C5-oxyalkyl, halogen, $-NH_2$ or-OH and the counter ion $X^+$ is selected from alkali metal or alkaline earth metal ions, $(R6)_4P^+$, $(R6)_4N^+$, wherein R6 is C1-C10-alkyl or C5-C10-aryl;
their use in the detection of mercury cations, a testkit therefore and the use of the testkit as well as precursor compounds.

## Description

Metal ions play critical roles in many physiological and pathological processes in living organisms but heavy metals such as mercury, which have no known vital or beneficial effect on organisms, and their accumulation over time in the bodies of animals and human beings can cause serious illness. Mercury is one of the most ubiquitous non-biodegradable hazardous pollutants with its various existing forms widely distributed in the ecosystem.[1] The toxicity of the mercury dication $Hg^{2+}$ is related to the fact that biological ligands (i.e. proteins/enzymes) can coordinate mercury preferably over essential metals due to its high affinity to S-functional groups in proteins (i.e. the amino acids cysteine and methionine).[2] Inorganic mercury ($Hg^{2+}$) can be converted into methylmercury by marine bacteria and then easily enter the food chain via large predatory fish, such as tuna and swordfish. When consumed by humans, methylmercury can easily pass through biological membranes due to its water solubility and concurrent lipophilicity. [3] Exposure to mercury ions even at low concentration can cause serious human health problems resulting in significant damage to the kidneys, heart, brain, stomach, intestines, mitosis impairment and permanent damage of the central nervous system[4] including the so called Minamata disease[5]. Also some people fear that mercury might be lost from of amalgam inlays frequently used in the last century in dental treatments. Given these environmental and biological concerns, much effort has been dedicated to develop a rapid, cost effective, easy to use, sensitive and selective method for detecting $Hg^{2+}$ ions to be used in environmental and biological milieus.[6] Only a test fulfilling all the criteria listed above would be able to help preventing the harmful effects of mercury released into the environment on the world population; in particular in third world countries.

The object of the present invention thus was the provision of a $Hg^{2+}$ test, which overcomes the disadvantages of presently available methods and is selective, sensitive, easy to use and cost effective.

The problem was solved by nickel complexes according to claim 1, their use as outlined in claims 5-8, a testkit as disclosed in claim 10 and its use as well as by precursor compounds according to claim 14.

In other words, the problem was solved by nickel complex according to formula I, especially for use in detection of $Hg^{2+}$ ions,

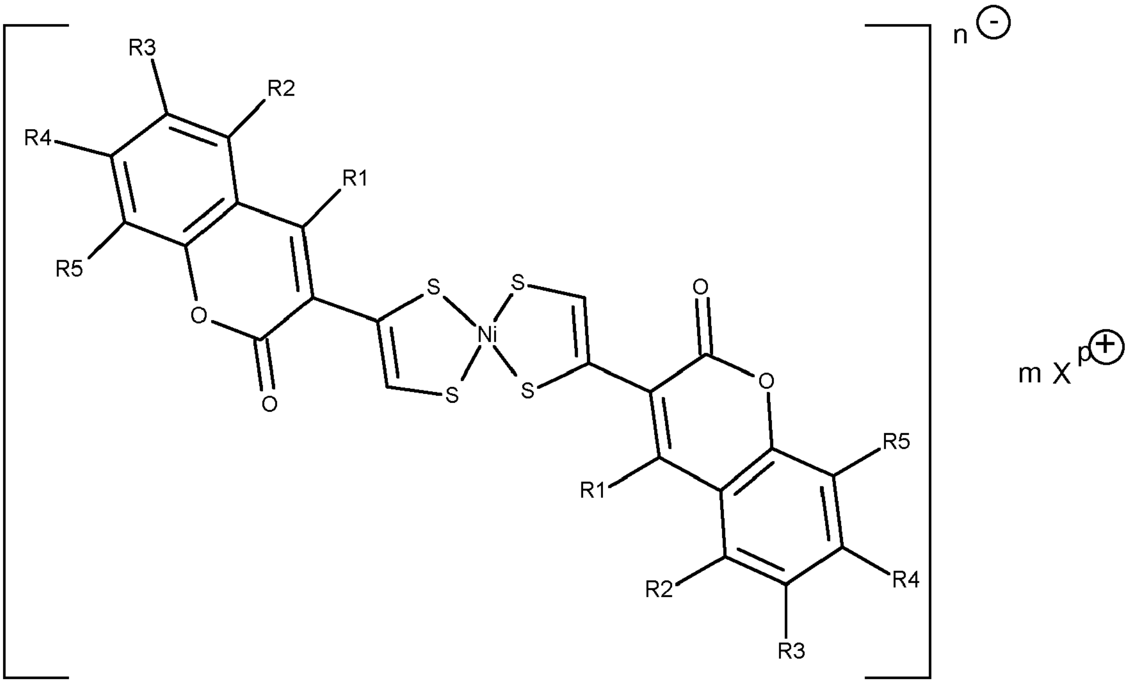

I

wherein

nickel has the oxidation state +II or +III,

n, m are each 1 or 2,

each group R1-R5 is independently selected from hydrogen, C1-C5-alkyl, C1-C5-oxyalkyl, halogen, $-NH_2$ or -OH and the counter ion $X^+$ with p = 1 or 2 is selected from alkali metal ions (preferably $Na^+$ or $K^+$), alkaline earth metal ions (preferably $Ca^{2+}$, $Mg^{2+}$),$(R6)_4P^+$, $(R6)_4N^+$, wherein R6 is C1-C10-alkyl or C5-C10-aryl.

In one embodiment, both groups R1 are each hydrogen and each of the groups R2-R5 is independently selected

from hydrogen or C1-C5-alkyl. Preferably each group R1-R5 is hydrogen.

For at least one residue of R1-R5 being C1-C5-alkyl, the alkyl group(s) is/are preferably selected from methyl (Me), ethyl (Et), n-propyl ($^{n}$Pr) or iso-probyl ($^{i}$Pr), n-butyl ($^{n}$Bu)or tert-butyl ($^{t}$Bu). If at least one residue of R1-R5 is C1-C5-oxyalkyl, it is preferably methoxy or ethoxy, most preferred methoxy. For at least one residue of R1-R5 being halogen, fluoride, chloride, bromide or iodide are preferred. In one embodiment, at least one C1-C5-alkyl group is present for R1-R5, which has at least one further substituent selected from -OH or -$NH_2$, preferably -OH, most preferably located at the opoosite end of the alkyl chain (opposite to the coumarine basic structure).

Table 1 shows preferred substituents for the Nickel complexes according to formula I:

**Table 1**

| | | | | | | | | | | | | |
|---|---|---|---|---|---|---|---|---|---|---|---|---|
| R1 | H | Me | Et | OH | | | | | | | | |
| R2 | H | Me | Et | OH | OMe | | | | | | | |
| R3 | H | Me | Et | OH | OMe | $^{i}$Pr | | $NH_2$ | F | Cl | Br | I |
| R4 | H | Me | | | OMe | -$CH_2OH$ | | $NH_2$ | F | Br | | |
| R5 | H | Me | | OH | | | $^{t}$Bu | | | Cl | | |

It is also preferred that the counter ion $X^+$ is $Ph_4P^+$.

Regarding the oxidation state of the metal, +III is preferred; resulting in n being 1, m being 1 and p being 1. The values for n and the product of the integers m and p preferably correspond to each other in that the nickel complex is neutrally charged to the outside. In other words: if n is 2, i.e. if the oxidation state of the nickel ion is +II, the product of m and p is 2 as well, which results in m being 1 and p being 2 or m being 2 and p being 1. p being 1 and m being 2 especially applies to all doubly charged ions (=alkaline earth metal ions mentioned above) as counter ion. If n is 1, m and p are each 1. This constellation especially applies to all singly charged ions mentioned above.

The residues R6 in $(R6)_4P^+$, $(R6)_4N^+$ are defined above as C1-C10-alkyl or C5-C10-aryl. Furthermore, for R6 being alkyl, a selection from the group of C1-C5-alkyl is preferred. For R6 being aryl, a selection from the group of C5-C7-aryl is preferred and the most preferred R6 aryl residue is phenyl. Each R6 can be selected independently from the others. Preferably, all residues R6 within one $(R6)_4P^+$, $(R6)_4N^+$ are are identical.

In one embodiment, each aryl group R6 may have further substituents, preferably at least one further substituent, most preferred just one further substituent per aryl residue. This/These further substituent(s) may differ between the four R6 residues but identical further substituent(s) are preferred. Said at least one or just one further substituent per aryl residue is preferably selected from C1-C3-alkyl and is most preferred methyl ($CH_3$).

One most preferred embodiment is related to a Nickel(III)complex as shown in formula Ia, which is synonymously called the coumarin bearing nickel-dithiolene complex $[Ni(S_2C_2Hcumrn)_2][Ph_4P]$:

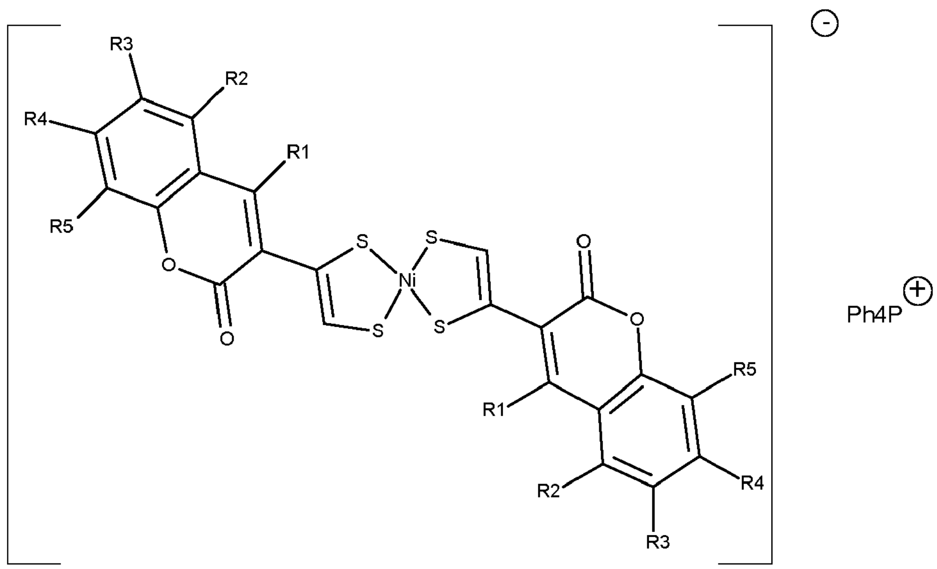

**Ia**

There are some examples of fluorescent probes having been developed for the detection of $Hg^{2+}$ because their high sensitivity and operational simplicity appeared to be most suitable.[7] But most of them possess absorbance and emission activity in the ultraviolet visible (UV-Vis) light range, which limits their applicability for $Hg^{2+}$ sensing in living systems since the light in this wavelength region is well absorbed by biomolecules leading to unfavorable signal-to-noise ratios.[8] Although other traditional analytical techniques including atomic absorption spectroscopy, atomic fluorescence spectrometry,[9] dispersive liquid-liquid microextraction,[10] inductively coupled plasma atomic emission spectrometry, electrochemical sensing, and the use of piezoelectric quartz crystals provide good sensitivity they do require expensive equipment, quite often tedious sample treatment is necessary and they may be completely unsuitable for on-site real time *in-situ* detection of metal ions.[2,11] Alternatively, applying absorbance spectroscopy in the NIR region to heavy metal ion detection has a significant advantage over other common analytical methods. First of all it is simple and cost effective. Paramount, however, is the fact, that the near-infrared (NIR) region of around 750-1400 nm possesses significant advantages including minimal interfering absorption from biological samples, low scattering and deep penetration into tissues.[8b, 12]

There are, only few examples in the literature in which sulfur functional groups are used in sensors, markers or indicators for binding the target.[13] A very interesting molecule used for the detection of lead[14] is in fact a precursor for a ditholene ligand.

All Nickel complexes according to formula I, especially the coumarin bearing nickel-dithiolene complex $[Ni(S_2C_2Hcumrn)_2][Ph_4P]$ according to formula Ia [later on also called (3)], possess an absorbance in the NIR region (~960 nm), which exhibits high sensitivity and selectivity toward $Hg^{2+}$ ions even in the presence of various background competitive metal ions ($Ag^+$, $Al^{3+}$, $Ca^{2+}$, $Cd^{2+}$, $Co^{2+}$, $Cu^{2+}$, $Fe^{3+}$, $K^+$, $Li^+$, $Mg^{2+}$, $Mn^{2+}$, $Na^+$, $Pb^{2+}$, $Sn^{2+}$ and $Zn^{2+}$). Hence, simple UV-Vis spectroscopy can be used with this indicator complex. It is, however, even possible to observe a distinct color fading of the solution with the naked eye; i.e. the cheapest optical method imaginable can be applied for the detection of $Hg^{2+}$ using these novel molecules.

Below, $[Ni(S_2C_2Hcumrn)_2][Ph_4P]$ (3) is described exemplarily as representative for all compounds of formula I.

For the total synthesis of the inventive compounds, especially the coumarin based nickel-dithiolene complex $[Ni(S_2C_2Hcumrn)_2][Ph_4P]$, a general strategy for the synthesis of unsymmetrically substituted ene-1,3-dithiolate (dithiolene) ligands was applied followed by metalation of the free (i.e. de-protected) dithiolate with simple nickel(II) chloride[15]

Scheme 1 shows the synthesis route for $[Ni(S_2C_2Hcumrn)_2][Ph_4P]$ as exemplary compound.

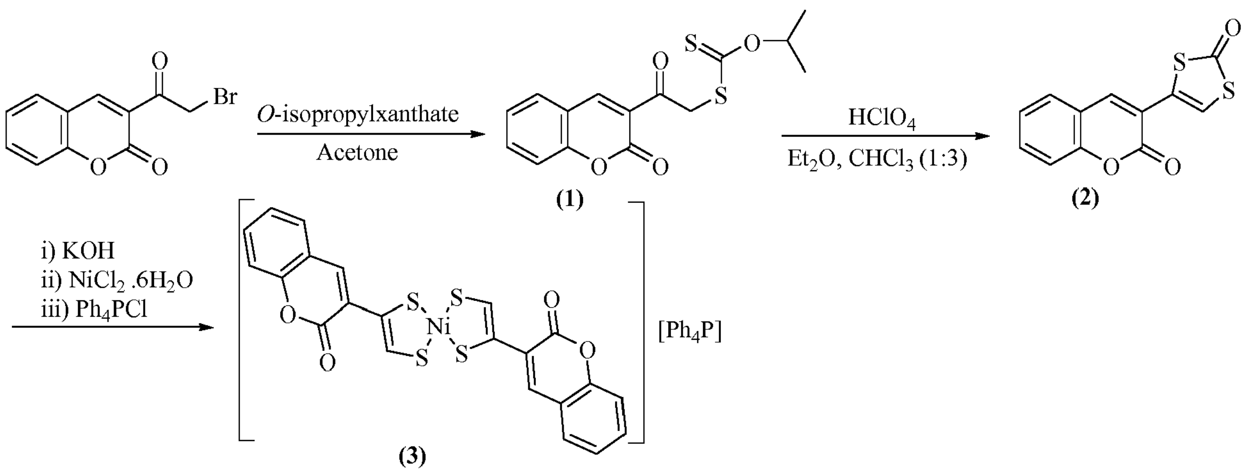


***Scheme 1***. Synthetic route to and molecular structure of the $[Ni(S_2C_2Hcumrn)_2][Ph_4P]$ complex **(3)** starting from a commercially available coumarin-bromo-ketone.

The ligand precursor 4-(coumarin) 1,3-dithiol-2-one **(2)** was obtained from a reaction between commercially available 3-(bromoacetyl) coumarin and an O-alkylxanthate salt affording intermediate **(1)**, which is then subjected to acid catalyzed ring closure. Crystallization of the crude products **(1)** and **(2)** by slow evaporation from chloroform yielded needle shaped crystals of off-white and light yellow color, respectively. X-ray single-crystal structural characterization of intermediate **(2)**, crystallized in the triclinic space group *P-1*, explains the stability of the compound revealing intra- and intermolecular hydrogen bonds in the crystal lattice involving the carbonyl-O with both dithiolene and aromatic hydrogen atoms and dithiolene-S with another aromatic hydrogen atom (Figure 1).

Treatment of **(2)** with 2.5 equivalent potassium hydroxide[15a] in methanol afforded the soluble disodium salt

of the dithiolate dianion, which was then allowed to react with $NiCl_2 \cdot 6H_2O$ dissolved in methanol. The monoanionic salt **[3]**- was precipitated from the dark brown reaction mixture using tetraphenylphosphonium chloride as bulky cation source (Scheme 1). Recrystallization of the crude complex from an acetonitrile:diethyl ether mixture at room temperature yielded the pure salt as a dark red-brown crystalline solid. The chemical structure of the target **(3)** was confirmed by $^{13}C$ NMR, elemental analysis, IR and MALDI/TOF mass spectrometry. Despite using a Ni(II) salt as precursor for the preparation of **(3)** the formal charge of the metal complex $[Ni(S_2C_2Hcumrn)_2]$ with distorted square planar geometry was presumed to be -1 according to similar complexes described in the literature.[15b-d, 16] This was confirmed by measuring the magnetic moment of the complex **(3)** via the Evan's NMR method.[17] As the reaction was carried out in air, molecular oxygen is the most likely oxidant for the oxidation from Ni(II) to Ni(III). The obtained complex appears to be oxygen and moisture insensitive as even after prolonged standing in air no changes of the sample have been observed.

Compounds for which 4-(coumarin) 1,3-dithiol-2-one **(2)** is shown above exemplarily, are also part of the present invention. In other words, compound according to formula II, which are especially usable in the synthesis of compounds of formula I,

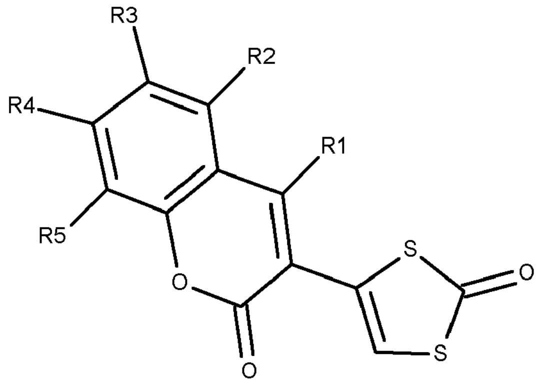

**II**

wherein R1-R5 have the same meaning as indicated above in the context of formula I, are also part of the present invention. The preferred variants of R1-R5 for formula II also correspond to those mentioned above for formula I.

The optical properties of the coumarin based nickel-dithiolene complex **(3)** were investigated by UV-Vis spectroscopy over a wide range. Interestingly, this specific complex is soluble in many nonpolar and polar organic solvents but insoluble in water. The exchange of the bulky counter cation $[Ph_4P^+]$ for a smaller one (i.e. $Bu_4N^+$, $K^+$, $Na^+$) increases the solubility significantly but lowers the yield from its synthesis as the complex now becomes water soluble. The coumarin part attached to the nickel dithiolene core has a large effect on both the position of electronic absorbance maxima and the solubility of the relevant dye (for instance when compared with naphthalin). It was found that **(3)** gave a green colored solution in acetonitrile with an absorbance maximum at 951 nm (Figure 2). The NIR absorption maximum, observed for this complex, is a function of both extensive electron delocalization within the dithiolene ring system, and the interaction of this delocalized system with available d-orbitals on the central metal.[18] The addition of $Hg^{2+}$ ions to this solution of **(3)** results in a rapid color change of the solution from green to colorless (Figure 2, inset; green colour only apparent in Figure 2 as shade of grey) ending with no absorbance at 951 nm or anywhere near. Apparently complex **(3)** can serve as a visual indicator for the presence of $Hg^{2+}$ ions, which is a great advantage over other analytical techniques because with this nickel complex $Hg^{2+}$ can be detected even using simple eyesight. To determine the requirements for a quantitative interaction of the nickel complex with the target ion, absorbance titrations of **(3)** with $Hg^{2+}$ ions were carried out. When $Hg^{2+}$ was added in distinct portions to the solution of **(3)** (50 μM), a gradual decrease of the absorbance at 951 was observed (Figure 2).

As shown in Figure 3, a continuous decrease of the absorbance at 951 nm was induced by increasing concentrations of $Hg^{2+}$ in the investigated sample solutions (0 to 0.2 mM; i.e. a fourfold excess of $Hg^{2+}$ with respect to the nickel complex). In the range of 0 to 0.15 mM $Hg^{2+}$ concentrations together with the 50 μM concentration of complex an almost linear dependency of the absorbance at 951 nm on the concentration of $Hg^{2+}$ was found. This means that the coumarin-dithiolene nickel complex can even be used for a photometric determination of $Hg^{2+}$ concentrations given that suitable experimental setups are chosen. The detection limit of complex **(3)** as an indicator for $Hg^{2+}$ was determined from the plot of the normalized absorbance as a function of the $Hg^{2+}$ concentrations.[19] It was found that **(3)** in 50 μM concentration had a detection limit of 0.13 mM for free $Hg^{2+}$. The minimal concentration of complex **(3)** detectable by UV-Vis spectroscopy and bare eye was found to be approximately 5 μM which accordingly can be used for detecting free $Hg^{2+}$ down to a concentration of 13.0 μM.

Thus, the qualitative and also the quantitative detection of $Hg^{2+}$ can be done by colorimetry or by photometry, especially by UV/Vis-spectroscopy.

For any excellent probe an inevitable requirement is a high selectivity for the target. The effects on the indicator solution of metal ions commonly found in the environment or with similar chemical properties as $Hg^{2+}$ such as $Ag^{+}$, $Al^{3+}$, $Ca^{2+}$, $Cd^{2+}$, $Co^{2+}$, $Cu^{2+}$, $Fe^{3+}$, $K^{+}$, $Li^{+}$, $Mg^{2+}$, $Mn^{2+}$, $Na^{+}$, $Pb^{2+}$, $Sn^{2+}$ and $Zn^{2+}$ were investigated by absorbance spectroscopy of the sensor solution after addition of each representative metal ion individually (Figure 5) and compared with $Hg^{2+}$. The color of the solution and the absorbance band remained unchanged upon addition of 4 equivalents of $Al^{3+}$, $Ca^{2+}$, $Cd^{2+}$, $Co^{2+}$, $K^{+}$, $Li^{+}$, $Mg^{2+}$, $Mn^{2+}$, $Na^{+}$, $Pb^{2+}$, $Sn^{2+}$, and $Zn^{2+}$ to the sensor solution as shown in Figures 4 and 5. Upon addition of 4 equivalents of $Fe^{3+}$ and $Cu^{2+}$ ions the absorbance maxima underwent a hypsochromic shift by 116 nm accompanied by a color change, which looks more like a fading to the bare eye. For $Ag^{+}$ ions two new peaks were observed (at 715 nm and 920 nm) and a color change to light brown upon addition of 4 equivalents. As the addition of $Hg^{2+}$ to the solution of **(3)** led to a minimum absorbance and a complete discoloring of the solution, probe **(3)** can colorimetrically discriminate $Hg^{2+}$ from all other tested metal ions with a characteristic NIR signature and easily even with the bare eye.

Notably, the tested ions might be added dissolved in any solvent, even water, without interfering with the optical properties of the indicator. To further explore the utility of probe **(3)** as an ion-selective indicator for $Hg^{2+}$, the selectivity studies were extended to different mixtures of environmentally relevant metal ions (Figure 6). The results clearly illustrate that the novel probe exhibits a very high selectivity toward $Hg^{2+}$ ions even in the presence of various background competitive metal ions and metal ion mixtures such as $Ag^{+}$, $Al^{3+}$, $Ca^{2+}$, $Cd^{2+}$, $Co^{2+}$, $Cu^{2+}$, $Fe^{3+}$, $K^{+}$, $Li^{+}$, $Mg^{2+}$, $Mn^{2+}$, $Na^{+}$, $Pb^{2+}$, $Sn^{2+}$ and $Zn^{2+}$ with a distinct discoloration of the solution. It was shown that the coumarin based nickel-dithiolene complex **(3)** can be utilized as an $Hg^{2+}$-selective indicator with evident sensitivity for $Hg^{2+}$ even in the presence of 4 equivalents of background $Cu^{2+}$, $Pb^{2+}$, and $Ag^{+}$ ions which were presumed to be potentially potent competitors.

In conclusion a facile and straightforward pathway for the synthesis of a new $Hg^{2+}$ indicator in form of a distorted square planar nickel-bisdithiolene complex **(3)** was developed. The probe exhibits high sensitivity and selectivity toward $Hg^{2+}$ ions even in the presence of various potentially competitive background metal ions. Aside from its very convincing selectivity, the synthetic complex **(3)** presented here is also superior to many previously discovered mercury sensors because of its synthetic simplicity and the low economical costs of its precursors (making it attractive for commercial use). In addition, the sensor demonstrated a colorimetric behavior toward $Hg^{2+}$ ions as evidenced by perceptible color changes of the solutions from green to colorless. Thus, compounds of formula I, especially **(3)** are usable in, for example, portable devices and test strips for "naked-eye" detection of $Hg^{2+}$.

Overall, the present invention is also related to the use of a Nickel complex as described above, for detection of $Hg^{2+}$ ions. Preferably, the detection of $Hg^{2+}$ ions happens based on the use of samples, wherein the sample is selected from food samples, ground samples, water samples, biological or organic samples. The biological or organic sample(s) are selected from bone samples, tissue samples, cell samples and tooth/dental samples

It goes without saying that the wording "detection" comprises qualitative as well as quantitative detection. In one embodiment, the detection is carried out in liquid phase. In another embodiment, the detection is done with the compound according to formula I being immobilized on a solid carrier (e.g. a test strip). In one very simple embodiment, the compound according to formula I can for example be soaked up by paper and then be used like a pH test strip.

Thus, a testkit for detection of $Hg^{2+}$ ions comprising a Nickel complex as described above is also within the focus of the present invention. Said testkit can be used for qualitative and/or quantitative detection of $Hg^{2+}$ ions. As already explained above with respect to the general use of the inventive compounds, the testkit is used for qualitative and/or quantitative detection of $Hg^{2+}$ ions in samples, preferably in food samples, ground samples, water samples, biological or organic samples.

In one embodiment, a stock solution of the complex and a spectrophotometer can be used for exact quantitative measurements. For qualitative tests nothing is needed except the compound in solid or dried or dissolved state. Regarding the preparation of solutions, any suitable organic solvent or water can be used. As organic solvents, preferably polar organic solvents can be used, more preferred aprotic polar organic solvents, for example acetonitrile. "Polar" means organic solvents having a $logK_{OW}$ between -1,0 and +2,0, preferably between -0,5 and +1,8. Besides acetonitrile ($logK_{OW}$ -0.34), alcohols such as ethanol, acetone, ethyl acetate, tetrahydrofuran are also usable as polar organic solvent. Mixtures of these solvents as well as mixtures with water can also be used.

**Description of Figures**

***Fig. 1a:*** Crystal structure of 4-(coumarin) 1,3-dithiol-2-one **(2)**. Ellipsoids are shown with 50% probability;

***Fig. 1b:*** intra and inter molecular hydrogen bonds in **(2)**;

***Fig. 2:*** absorption spectra of **(3)** (c = 5 x $10^{-5}$ M) in acetonitrile with added $Hg^{2+}$ (0.0, 0.5, 1.0, 1.5, 2.0, 2.5, 3.0, 3.5, 4.0, 4.5, 5.0, 5.5, 6.0 mole equivalents). Inset: the color of a solution of **(3)** in the absence and presence of $Hg^{2+}$;

***Fig. 3:*** absorbance titration of **(3)** (c = 50 μM) at 951 nm in acetonitrile upon addition of different portions of $Hg^{2+}$ (final $Hg^{2+}$ concentration: 0.0, 0.025, 0.05, 0.075, 0.10, 0.125, 0.15, 0.175, 0.20, 0.225, 0.25, 0.275, 0.30 mM) with gradual color change;

***Fig. 4:*** color stability or change of **3** (50 μM) in acetonitrile observed upon adding 4 mole equivalents of different metal ions;

***Fig. 5:*** UV/Vis absorption spectra of **3** (50 μM) in acetonitrile upon addition of salts (4.0 mole equiv.) of $Ag^+$, $Al^{3+}$, $Ca^{2+}$, $Cd^{2+}$, $Co^{2+}$,$Cu^{2+}$, $Fe^{3+}$, $K^+$, $Li^+$, $Mg^{2+}$, $Mn^{2+}$, $Na^+$, $Pb^{2+}$, $Sn^{2+}$, $Zn^{2+}$ and $Hg^{2+}$;

***Fig. 6:*** selectivity experiments of (**3**) (50 μM) in acetonitrile toward $Hg^{2+}$ in methanol (4 equiv.) over a mixture of environmentally relevant metal ions.

Below, Examples and experimental conditions are described:

**Experimental Section**

**Materials and methods:** All materials were purchased from Sigma Aldrich and abcr and were used without further purification. NMR measurements were recorded on a Bruker Avance II-300 MHz instrument. All samples were dissolved in deuterated solvents and Chemical shifts (δ) are given in parts per million (ppm) using solvent signals as the reference ($CDCl_3$ $^1H$: δ = 7.24 ppm; $^{13}C$: δ = 77.0 ppm) related to external tetramethylsilane (δ = 0 ppm). Coupling constants (J) are reported in Hertz (Hz) and splitting patterns are designated as s (singulet), d (doublet), t (triplet), q (quadruplet), quint (quintuplet), spt (septet), m (multiplet), dd (doublet of doublet). All infrared spectra were recorded (4000-400 $cm^{-1}$) on a Perkin-Elmer Fourier-Transform Infrared (FTIR) spectrophotometer as KBr pellets. Elemental analyses (C, H, N and S) were carried out with an Elementar Vario micro elemental analyzer. Mass spectra were measured on a Brucker microflex. matrix assisted laser desorption/ionization-(MALDI-TOF) spectrometer.

**General method for the UV-vis titration:**

For UV-vis titrations a stock solution of the $[Ph_4P][Ni(S_2C_2Hcumrn)_2]$ complex (**3**) was prepared (c = 5 x $10^{-5}$M) in $CH_3CN$ (25 °C). The MeOH solution of the cation to be tested was prepared using the respective chloride salt in a concentration of $10^{-2}$ M (25 °C). The spectra of the solutions or solution mixtures respectively were recorded on a Shimadzu 3600 UV-vis instrument. All measurements were made at room temperature (25 °C) in a quartz cuvettes (path length = 1 cm).

**Single-crystal X-ray diffraction:** Suitable single crystals of **1** and **2** were mounted on a thin glass fibre coated with paraffin oil. X-ray single-crystal structural data were collected at low temperature (170 k) using a STOE-IPDS 2T diffractometer equipped with a normal-focus, 2.4 kW, sealed-tube X-ray source with graphite-monochromated $Mo_{K\alpha}$ radiation (λ =0.71073 A). The program XArea was used for integration of diffraction profiles; numerical absorption correction was made with the programs X-shape and X-red32; all from STOE © 2010. The structures were solved by SIR92[20] and refined by full-matrix least-squares methods using SHELXL-2013[21]. The non-hydrogen atoms were refined anisotropically. The hydrogen atoms were refined isotropically on calculated positions using a riding model with their $U_{iso}$ values constrained to 1.5 $U_{eq}$ of their pivot atoms for terminal $sp^3$ carbon atoms and 1.2 times for all other carbon atoms. All crystallographic and structure-refinement data of **1** and **2** are summarized in Table **2**. Selected bond distances and angles are given in Tables **3** and **4**. All calculations were carried out using SHELXL-2013[21] and the WinGX system, Ver1.70.01[22].

**Table 2**: Crystal Data and Structure Refinement Parameters for **1** and **2**.

| parameters | **1** | **2** |
|---|---|---|
| empirical formula | $C_{15}H_{14}O_4S_2$ | $C_{12}H_6O_3S_2$ |
| formula weight | 322.38 | 262.29 |
| crystal system | Triclinic | Triclinic |
| space group | P-1 | P-1 |
| *a*, Å | 8.2023(16) | 6.7063(13) |

(continued)

| parameters | **1** | **2** |
|---|---|---|
| *b*, Å | 10.177(2) | 7.2111(14) |
| *c*, Å | 10.298(2) | 12.227(2) |
| $\alpha$, deg | 118.32(3) | 104.07(3) |
| $\beta$, deg | 94.42(3) | 93.64(3) |
| $\gamma$, deg | 99.51(3) | 112.61(3) |
| *V*, $Å^3$ | 734.3(3) | 521.28(18) |
| *Z* | 2 | 2 |
| *T*, K | 170(2) | 170(2) |
| $\lambda(Mo_{k\alpha})$,Å | 0.71073 | 0.71073 |
| $\mu$,$mm^{-1}$ | 0.375 | 0.500 |
| $D_{calcd}$,$g/cm^3$ | 1.458 | 1.671 |
| *F* (000) | 336 | 268 |
| reflections [$I>2\sigma(I)$] | 2901 | 2032 |
| unique reflections | 3927 | 2789 |
| measured reflections | 8808 | 5715 |
| $R_{int}$ | 0.0386 | 0.0892 |
| GOF on $F^2$ | 1.183 | 1.165 |
| $R_1[I>2\sigma(I)]$[a] | 0.0483 | 0.0792 |
| $R_w[I>2\sigma(I)]$[b] | 0.1163 | 0.2196 |
| $\Delta\rho$ max/min [e $Å^{-3}$] | 0.462, -0.442 | 0.522 , -0.526 |
| [a]: $R = \sum \|\|Fo\| - \|Fc\|\|/\sum \|Fo\|$; [b]: $R_w = [\sum\{w(F_o^2 - F_c^2)^2\}/\sum\{w(F_o^2)^2\}]^{1/2}$. | | |

**Synthesis and characterization:**

**Synthesis of O-isopropyl S-2-oxo-2-coumarine-ethylcarbonodithioate (1) :**

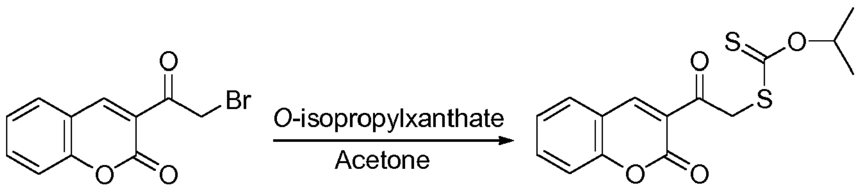


**(1)**

A solution of O-isopropylxanthate (0.366 g, 2.1 mmol) in anhydrous acetone (20 ml) was added slowly to a solution of 3-(Bromoacetyl) coumarin (0.53g, 2mmol) in anhydrous acetone (5 ml). The resulting solution was stirred for 2h and then filtered to remove KBr; the solvent was removed by a rotatory evaporator. The residue was suspended in 100 ml water and extracted with $CHCl_3$. The organic portions were combined, dried over $MgSO_4$ and concentrated on a rotary evaporator to afford 0.4932 g (76.5 %) of O-isopropyl S-2-oxo-2-coumarine-ethylcarbonodithioate as off white colored product. Crystallization of the crude product by slow evaporation from chloroform yielded the off-white colored needle shaped crystals. Crystal data and selected bond lengths (Å) and angles (°) for **1** are given in Tables 2 and 3. Yield: 76.5 % (0.4932 g). $^1H$ NMR (300 MHz, $CDCl_3$) $\delta$ (ppm): 1.39 (d, J = 6.04 Hz, 6H), 4.69 (s, 2H), 5.69 (m, J = 6.42 Hz,1H), 7.40 (dd, J = 7.93 Hz, 2H), 7.69 (d, J = 7.55 Hz, 2H), 8.55 (s, 1H). IR (KBr pellet): ($u/cm^{-1}$) = 3442, 3049, 2984, 2964, 2920, 2870, 1747, 1725, 1688, 1604, 1556, 1489, 1454, 1379, 1367, 1340, 1308, 1271, 1243, 1175, 1142, 1091, 1045, 996, 968, 924, 899, 872, 863, 788, 771, 763, 714, 637, 570, 447.

**Crystal data for 1**: $C_{15}H_{14}O_4S_2$; $M_r$=322.38; triclinic, space group *P-1*; *a*=8.2023(16), *b*=10.177(2), *c*=10.298(2) Å; $\alpha$=118.32(3),$\beta$=94.42(3),$\gamma$=99.51(3) °;V=734.3(3)$Å^3$; *Z*=2; *T*=170(2) K; $\lambda(Mo_{k\alpha})$=0.71073 Å; $D_{calcd}$= 1.458 $gcm^{-3}$ ; $\mu$=0.375 $mm^{-1}$. A total of 8808 reflections were collected, of which 3927 were unique ($R_{int}$=0.0386); $R_1$=0.0483, $wR_2$=0.1163 for $I>2\sigma(I)$.

**Synthesis of 4-coumarine-1,3-dithio-2-one (2):**

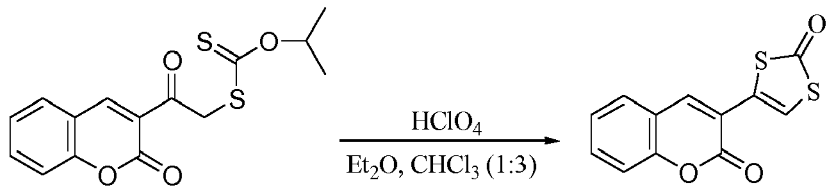


**(2)**

O-isopropyl-S-2-oxo-2-coumarine-ethylcarbonodithioate (0.49g, 1.53mmol) was suspended in 16 ml $Et_2O$:$CHCl_3$ 1:3. Perchloric acid (70 %, 0.92 ml, 15.3 mmol ) was added and the mixture PVA-24464 EP FASSUNG was heated up to 65 °C. During the heating period all starting material was dissolved. The mixture was kept at 65 °C for additional 6 h and a pale yellow solid precipitated during stirring. Then the precipitate was filtered off, washed with $Et_2O$ (5 ml) and dried under vacuum. The title compound could be obtained as cream-coloured powder. Crystallization of the crude product **(2)** by slow evaporation from chloroform yielded off-white colored needle shaped crystals. Crystal data and selected bond lengths (Å) and angles (°) for **(2)** are given in Tables 2 and 4. Yield: 84.7 % (0.34 g). $^1$H NMR (300 MHz, $CDCl_3$) δ (ppm): 8.07 (s, 1 H), 7.66 (s, 1 H), 7.58 (m, J = 6.80 Hz, 2H), 7.38 (m, J = 6.80 Hz, 2H). $^{13}$C-NMR(300 MHz, $CDCl_3$) δ (ppm): 190.54, 158.21, 152.64, 139.78, 132.61, 128.32, 127.35, 125.12, 120.93, 119.28, 118.58, 116.59. IR (KBr pellet): (*u*/cm$^{-1}$) = 3434, 3099, 3023, 1709, 1643, 1602, 1560, 1486, 1449, 1371, 1292, 1261, 1215, 1124, 1096, 957, 930, 884, 850, 795, 762, 736, 652, 623, 561, 525, 452, 433. Elemental analysis: calcd. For $C_{12}H_6O_3S_2$: C, 54.95; H, 2.31; S, 24.45; Found: C, 53.83; H, 2.42; S, 24.13.

**Crystal data for 2:** $C_{12}H_6O_3S_2$; $M_r$= 262.29; triclinic, space group *P-1*; *a*=6.7063(13), *b*=7.2111(14), *c*=12.227(2) Å; α=104.07(3),β=93.64(3),γ=112.61(3) °;*V*=521.28(18) Å$^3$; *Z*=2; *T*=170(2) K; λ($Mo_{k\alpha}$)=0.71073 Å; $D_{calcd}$= 1.671 gcm$^{-3}$ ; μ=0.500 mm$^{-1}$. A total of 5715 reflections were collected, of which 2789 were unique ($R_{int}$=0.0892); $R_1$=0.0792, $wR_2$=0.2196 for $I>2\sigma(I)$.

**Synthesis of [$Ph_4P$][$Ni(S_2C_2Hcumrn)_2$] (3):**

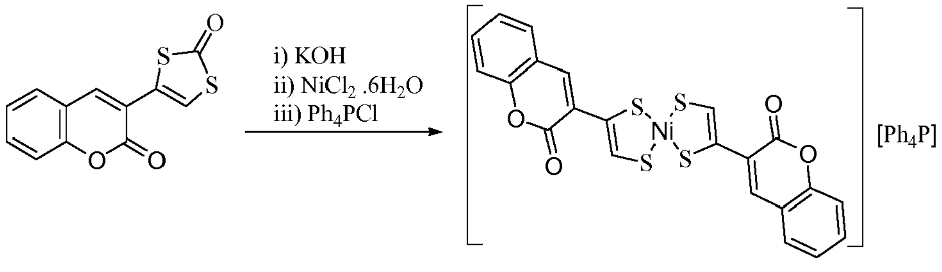


**(3)**

At room temperature, compound **2** (0.053 g, 0.2mmol) and KOH (0.028 g, 0.5mmol) were suspended in 10 ml dry methanol and stirred under nitrogen for 1 h, affording a red brown colored solution. A 3 ml methanol solution of $NiCl_2 \cdot 6H_2O$ (0.024 g, 0.1mmol) was added, and the resulting reaction mixture was stirred for 0.5 h. The solution turned to dark brown in color. Then a 3 ml methanol solution of $Ph_4PCl$ (0.075 g, 0.2mmol) was added under nitrogen atmosphere, and the reaction mixture was continuously stirred for 0.5 h. Then it was concentrated to about 5 ml, affording a dark red-brown precipitate. The product was obtained by filtration under nitrogen and washed with dry methanol. Yield: 47.3 % (0.082 g). IR (KBr pellet): (*u*/cm$^{-1}$) = 3423, 3058, 2357, 2205, 1714, 1648, 1607, 1554, 1483, 1457, 1437, 1285, 1256, 1229, 1186, 1151, 1108, 1071, 996, 946, 926, 870, 811, 755, 723, 689, 621, 571, 527, 448. Elemental analysis: calcd. For $C_{46}H_{32}NiO_4PS_4$: C, 63.75; H, 3.72; S, 14.80; Found: C, 62.97; H, 3.56; S, 14.43. MALDI-TOF MS: calcd. for $C_{22}H_{12}NiO_4S_4$ [M]$^-$ 525.9, Found: 525.703.

**Magnetic moment measurements via the Evan's NMR method[17]:**

The magnetic moment of compound (**3**) was determined using the following equation (Evan's method) and an NMR experiment in $CD_3CN$ at 300 K with a Bruker Avance II-300 MHz spectrometer and a 5mm Wilmad Coaxial Insert

NMR tube (capillary tube).

$$\mu_{eff} = \frac{A}{\sqrt{\frac{c}{T.\Delta ppm}}}$$

$\mu_{eff}$ = magnetic moment; *c* = Concentration in mol/L; *T* = Temperature [K]; *Δppm* = difference of chemical shift by paramagnetic substances and *A* = constant which can be solved by calibrating a paramagnetic substances with known $\mu_{eff}$ values.

The NMR tube was filled with 0.5 ml 0.0032077 M solution of compound (**3**) prepared using a mixture of $CD_3CN$ (475 $\mu L$) and $^tBuOH$ (25 $\mu L$) as solvent. The capillary tube was filled with 0.5 ml of the same solvent mixture ($CD_3CN$: 475 $\mu L$; $^tBuOH$: 25 $\mu L$). Then, the capillary was placed into the NMR tube in such a manner that the solvent in the NMR tube should just cover the capillary so that the tube floats freely from the sides of the NMR tube. The idea is that it shouldn't be like a sinker on the bottom of the NMR tube but it shouldn't float off the bottom too far either. And finally the $^1H$ NMR spectrum was measured for this sample at 300 K. The difference of the chemical shift of the methyl protons of $^tBuOH$ due to the presence of a paramagnetic substance (complex **3**) was found to be 1.31 at 300 K.

For calibration a 0.5 ml solution of $Cu(NO_3)_2$ (*c* = 0.0107705 M) using $CD_3CN$ (475 $\mu L$) and $^tBuOH$ (25 $\mu L$) as solvent was placed in the NMR tube and the capillary tube was filled with 0.5 ml of same solvent mixture ($CD_3CN$: 475 $\mu L$; $^tBuOH$: 25 $\mu L$). Then, the capillary was placed into the NMR tube in the same way described above and $^1H$ NMR was measured at 300 K. The difference of the chemical shift of the methyl protons of $^tBuOH$ was found to be 0.93 at 300 K.

A was calculated to be 0.012050609 at 300 K by calibration with $Cu(NO_3)_2$ (*c* = 0.0107705 M, *Δppm* = 0.93), which is known to be paramagnetic with a $\mu_{eff}$ value of 1.94[23]. The magnetic moment of compound (**3**) was determined to be 4.22 from the Evan's equation:

$$\mu_{eff} = \frac{0.012050609}{\sqrt{\frac{0.0032077\ M}{300.15\ K \times 1.31}}} = 4.22$$

The whole experiment was repeated three times and the average $\mu_{eff}$ value was eventually found to be 4.17.

Since the magnetic moment is a function only of the number of unpaired electrons (theoretically: $\mu_{eff} = \sqrt{n(n+2)}$; *n*: number of unpaired electrons), the present distorted square planar $[Ph_4P][Ni(S_2C_2Hcumrn)_2]$ complex has to be described as a formal Ni(III) system with its magnetic moment corresponding to approximately three unpaired electrons.

**Table 3:** Selected Bond lengths (Å) and angles (°) for **1**.

| | | | |
|---|---|---|---|
| C(1)-C(9) | 1.352(4) | C(1)-C(2) | 1.426(4) |
| C(2)-C(7) | 1.397(4) | C(2)-C(3) | 1.399(5) |
| C(3)-C(4) | 1.390(4) | C(4)-C(5) | 1.383(5) |
| C(5)-C(6) | 1.383(5) | C(6)-C(7) | 1.377(4) |
| C(7)-O(1) | 1.371(4) | C(8)-O(2) | 1.203(4) |
| C(8)-O(1) | 1.387(4) | C(8)-C(9) | 1.457(4) |
| C(9)-C(10) | 1.484(4) | C(10)-O(3) | 1.217(4) |
| C(10)-C(11) | 1.507(4) | C(11)-S(1) | 1.787(3) |
| C(12)-O(4) | 1.331(4) | C(12)-S(2) | 1.629(3) |
| C(12)-S(1) | 1.752(3) | C(13)-O(4) | 1.466(4) |
| C(13)-C(14) | 1.503(5) | C(13)-C(15) | 1.516(5) |
| | | | |
| C(9)-C(1)-C(2) | 121.9(3) | C(7)-C(2)-C(3) | 118.8(3) |
| C(7)-C(2)-C(1) | 117.9(3) | C(3)-C(2)-C(1) | 123.3(3) |

(continued)

| | | | |
|---|---|---|---|
| C(4)-C(3)-C(2) | 119.6(3) | C(5)-C(4)-C(3) | 120.1(3) |
| C(4)-C(5)-C(6) | 121.1(3) | C(7)-C(6)-C(5) | 118.7(3) |
| O(1)-C(7)-C(6) | 117.8(3) | O(1)-C(7)-C(2) | 120.5(3) |
| C(6)-C(7)-C(2) | 121.7(3) | O(2)-C(8)-O(1) | 115.8(3) |
| O(2)-C(8)-C(9) | 127.2(3) | O(1)-C(8)-C(9) | 117.1(3) |
| C(1)-C(9)-C(8) | 119.8(3) | C(1)-C(9)-C(10) | 118.0(3) |
| C(8)-C(9)-C(10) | 122.2(3) | O(3)-C(10)-C(9) | 119.4(3) |
| O(3)-C(10)-C(11) | 120.9(3) | C(9)-C(10)-C(11) | 119.7(3) |
| C(10)-C(11)-S(1) | 113.4(2) | O(4)-C(12)-S(2) | 127.9(2) |
| O(4)-C(12)-S(1) | 105.7(2) | S(2)-C(12)-S(1) | 126.42(18) |
| O(4)-C(13)-C(14) | 109.7(3) | O(4)-C(13)-C(15) | 104.9(3) |
| C(14)-C(13)-C(15) | 113.1(3) | C(7)-O(1)-C(8) | 122.8(2) |
| C(12)-O(4)-C(13) | 119.6(2) | C(12)-S(1)-C(11) | 102.47(15) |

**Table 4:** Selected Bond lengths (A) and angles (°) for **2**.

| | | | |
|---|---|---|---|
| C(1)-C(9) | 1.354(5) | C(1)-C(2) | 1.439(5) |
| C(2)-C(7) | 1.391(5) | C(2)-C(3) | 1.398(5) |
| C(3)-C(4) | 1.385(5) | C(4)-C(5) | 1.384(5) |
| C(5)-C(6) | 1.378(5) | C(6)-C(7) | 1.393(5) |
| C(7)-O(1) | 1.369(4) | C(8)-O(2) | 1.209(4) |
| C(8)-O(1) | 1.368(4) | C(8)-C(9) | 1.467(4) |
| C(9)-C(10) | 1.464(5) | C(10)-C(11) | 1.337(5) |
| C(10)-S(1) | 1.756(3) | C(11)-S(2) | 1.730(4) |
| C(12)-O(3) | 1.196(4) | C(12)-S(1) | 1.772(4) |
| C(12)-S(2) | 1.774(4) | | |
| | | | |
| C(9)-C(1)-C(2) | 121.4(3) | C(7)-C(2)-C(3) | 118.5(3) |
| C(7)-C(2)-C(1) | 117.9(3) | C(3)-C(2)-C(1) | 123.6(3) |
| C(4)-C(3)-C(2) | 119.7(3) | C(5)-C(4)-C(3) | 120.5(3) |
| C(6)-C(5)-C(4) | 121.1(3) | C(5)-C(6)-C(7) | 118.1(3) |
| O(1)-C(7)-C(2) | 120.5(3) | O(1)-C(7)-C(6) | 117.4(3) |
| C(2)-C(7)-C(6) | 122.1(3) | O(2)-C(8)-O(1) | 116.4(3) |
| O(2)-C(8)-C(9) | 126.4(3) | O(1)-C(8)-C(9) | 117.2(3) |
| C(1)-C(9)-C(10) | 122.1(3) | C(1)-C(9)-C(8) | 119.6(3) |
| C(10)-C(9)-C(8) | 118.3(3) | C(11)-C(10)-C(9) | 127.3(3) |
| C(11)-C(10)-S(1) | 115.2(3) | C(9)-C(10)-S(1) | 117.4(3) |
| C(10)-C(11)-S(2) | 119.5(3) | O(3)-C(12)-S(1) | 124.0(3) |
| O(3)-C(12)-S(2) | 124.1(3) | S(1)-C(12)-S(2) | 111.9(2) |

(continued)

| | | | |
|---|---|---|---|
| C(8)-O(1)-C(7) | 123.4(3) | C(10)-S(1)-C(12) | 97.19(17) |
| C(11)-S(2)-C(12) | 96.09(18) | | |

**References**

[1] a) E. M. Nolan, S. J. Lippard, Chem. Rev. 2008, 108, 3443-3480; b) H. H. Harris, I. J. Pickering, G. N. George, Science 2003, 301, 1203.

[2] M. Saleem, R. Abdullah, A. Ali, B. J. Park, E. H. Choi, I. S. Hong, K. H. Lee, Anal. Methods. 2014, 6, 3588-3597.

[3] a) A. Renzoni, F. Zino, E. Franchi, Environ. Res. 1998, 77, 68-72; b) S. Hardy, P. Jones, J. Chromatogr. A 1997, 791, 333-338.

[4] a) P. B. Tchounwou, W. K. Ayensu, N. Ninashvili, D. Sutton, Environ. Toxicol. 2003, 18, 149-175; b) J. Gutknecht, J. Membr. Biol. 1981, 61, 61-66.

[5] M. Harada, Crit. Rev. Toxicol. 1995, 25, 1-24.

[6] N. Lu, C. Shao, Z. Deng, Analyst 2009, 134, 1822-1825.

[7] a) Y.-K. Yang, K.-J. Yook, J. Tae, J. Am. Chem. Soc. 2005, 127, 16760-16761; b) X. Zhang, Y. Xiao, X. Qian, Angew. Chem. Int. Ed. 2008, 47, 8025-8029; c) B.-C. Ye, B.-C. Yin, Angew. Chem. Int. Ed. 2008, 47, 8386-8389; d) S. Yoon, E. W. Miller, Q. He, P. H. Do, C. J. Chang, Angew. Chem. Int. Ed. 2007, 46, 6658-6661; e) J.-S. Lee, M. S. Han, C. A. Mirkin, Angew. Chem. 2007, 119, 4171-4174; f) H. N. Kim, W. X. Ren, J. S. Kim, J. Yoon, Chem. Soc. Rev. 2012, 41, 3210-3244; g) A. P. de Silva, H. Q. N. Gunaratne, T. Gunnlaugsson, A. J. M. Huxley, C. P. McCoy, J. T. Rademacher, T. E. Rice, Chem. Rev. 1997, 97, 1515-1566.

[8] a) B. Zhu, W. Wang, L. Liu, H. Jiang, B. Du, Q. Wei, Sensor. Actuat. B-Chem. 2014, 191, 605-611; b) L. Yuan, W. Lin, K. Zheng, L. He, W. Huang, Chemical Society Reviews 2013, 42, 622-661.

[9] Z. Dong, X. Tian, Y. Chen, J. Hou, Y. Guo, J. Sun, J. Ma, Dyes. Pigments 2013, 97, 324-329.

[10] J. Liu, D. Wu, C. Duan, Y. Guan, Talanta 2013, 105, 87-92.

[11] a) X. Chen, S.-W. Nam, M. J. Jou, Y. Kim, S.-J. Kim, S. Park, J. Yoon, Org. Lett. 2008, 10, 5235-5238; b) J. Ni, Q. Li, B. Li, L. Zhang, Sensor. Actuat. B-Chem. 2013, 186, 278-285.

[12] Z. Guo, W. Zhu, M. Zhu, X. Wu, H. Tian, Chem. Eur. J. 2010, 16, 14424-14432.

[13] a) M.-H. Ha-Thi, M. Penhoat, V. Michelet, I. Leray, Org. Lett. 2007, 9, 1133-1136; b) L. Praveen, V. B. Ganga, R. Thirumalai, T. Sreeja, M. L. P. Reddy, R. L. Varma, Inorg. Chem. 2007, 46, 6277-6282; c) H. Wang, Y. Li, S. Xu, Y. Li, C. Zhou, X. Fei, L. Sun, C. Zhang, Y. Li, Q. Yang, X. Xu, Org. Biomol. Chem. 2011, 9, 2850-2855; d) N. Wanichacheva, S. Watpathomsub, V. S. Lee, K. Grudpan, Molecules 2010, 15, 1798-1810; e) J. Du, M. Hu, J. Fan, X. Peng, *Chem. Soc. Rev.* **2012**, *41*, 4511-4535.

[14] L. Marbella, B. Serli-Mitasev, P. Basu, Angew. Chem., Int. Ed. 2009, 48, 3996-3998, S3996/3991-S3996/3994.

[15] a) A. C. Ghosh, J. K. Reinhardt, M. K. Kindermann, C. Schulzke, Chem. Commun. 2014, 50, 10102-10104; b) O. Jeannin, R. Clérac, M. Fourmigué, Journal of the American Chemical Society 2006, 128, 14649-14656; c) T. Anjos, S. J. Roberts-Bleming, A. Charlton, N. Robertson, A. R. Mount, S. J. Coles, M. B. Hursthouse, M. Kalaji, P. J. Murphy, Journal of Materials Chemistry 2008, 18, 475-483; d) M. Fourmigue, J. N. Bertran, Chemical Communications 2000, 2111-2112.

[16] a) B. S. Lim, D. V. Fomitchev, R. H. Holm, Inorg. Chem. 2001, 40, 4257-4262; b) V. Madhu, S. K. Das, Inorg. Chem. 2008, 47, 5055-5070; c) L. N. Dawe, J. Miglioi, L. Turnbow, M. L. Taliaferro, W. W. Shum, J. D. Bagnato, L. N. Zakharov, A. L. Rheingold, A. M. Arif, M. Fourmigué, J. S. Miller, Inorg. Chem. 2005, 44, 7530-7539.

[17] a) D. F. Evans, J. Chem. Soc. 1959, 2003-2005; b) D. F. Evans, G. V. Fazakerley, R. F. Phillips, J. Chem. Soc. (A) 1971, 1931-1934; c) D. F. Evans, D. A. Jakubovic, J. Chem. Soc., Dalton Trans. 1988, 2927-2933; d) H. P. Fritz, K. E. Schwarzhans, J. Organomet. Chem. 1964, 1, 208-211; e) D. F. Evans, T. A. James, J. Chem. Soc., Dalton Trans. 1979, 723-726.

[18] Q. Fang, Y.-M. Sun, X.-Z. You, Chin. J. Chem. Phys. 1992, 1, 129.

[19] M. Shortreed, R. Kopelman, M. Kuhn, B. Hoyland, Anal. Chem. 1996, 68, 1414-1418.

[20] A. Altomare, G. Cascarano, C. Giacovazzo, A. Gualaradi, J. Appl. Crystallogr. 1993, 26, 343.

[21] G. M. Sheldrick, Acta Crystallogr. A 2008, 64, 112-122.

[22] L. Farrugia, J. Appl. Crystallogr. 1999, 32, 837-838.

[23] E. A. Boudreaux, D. J. Miller, Inorg. Nucl. Chem. Lett. 1966, 2, 59-61.

## Claims

1. Nickel complex according to formula I, especially for use in detection of $Hg^{2+}$ ions,

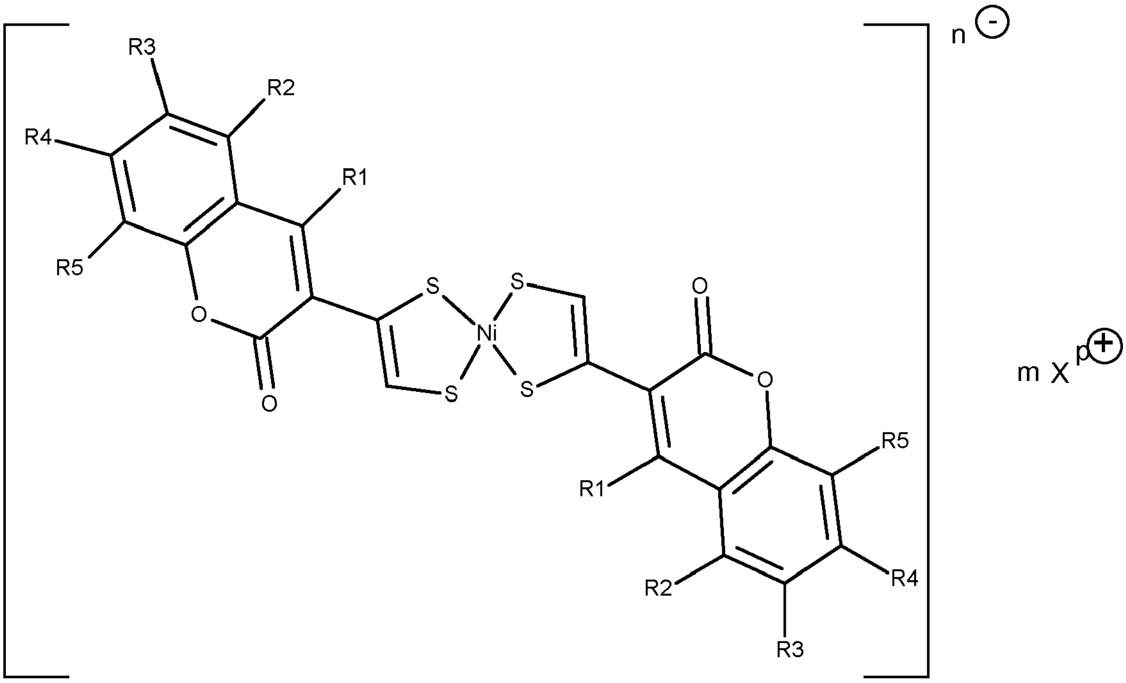

I

wherein
Nickel has the oxidation state II or III,
n, m are each 1 or 2, each group R1-R5 is independently selected from hydrogen, C1-C5-alkyl, C1-C5-oxyalkyl, halogen, $-NH_2$ or-OH and
the counter ion $X^+$ with p = 1 or 2 is selected from alkali metal or alkaline earth metal ions, $(R6)_4P^+$, $(R6)_4N^+$, wherein R6 is C1-C10-alkyl or C5-C10-aryl.

2. Nickel complex according to claim 1, wherein both groups R1 are each hydrogen and each of the groups R2-R5 is independently selected from hydrogen or C1-C5-alkyl.

3. Nickel complex according to claim 1 or 2, wherein each group R1-R5 is hydrogen.

4. Nickel complex according to any one of claims 1 to 3, wherein the counter ion $X^+$ is $Ph_4P^+$.

5. Nickel complex according to any one of claims 1 to 4, wherein Nickel has the oxidation state III, n is 1, m is 1 and p is 1.

6. Use of a Nickel complex according to any one of claims 1 to 5, for detection of $Hg^{2+}$ ions.

7. Use of the Nickel complex according to claim 6 for detection of $Hg^{2+}$ ions in samples, wherein the sample is selected from food samples, ground samples, water samples, biological or organic samples.

8. Use of the Nickel complex according to claim 7 for detection of $Hg^{2+}$ ions in samples, wherein the sample biological or organic are selected from bone samples, tissue samples, cell samples and tooth/dental samples

9. Use of the Nickel complex according to any one of claims 6 to 8, wherein the detection is qualitative and/or quantitative.

10. Use of the Nickel complex according to any one of claims 6 to 9, wherein the detection is carried out in liquid phase or immobilized on a solid carrier (test strip).

**11.** Testkit for detection of $Hg^{2+}$ ions comprising a Nickel complex according to any one of claims 1 to 5.

**12.** Use of the testkit according to claim 11 for qualitative and/or quantitative detection of $Hg^{2+}$ ions.

**13.** Use of the testkit according to claim 11 or 12 for qualitative and/or quantitative detection of $Hg^{2+}$ ions in samples, preferably in food samples, ground samples, water samples, biological or organic samples.

**14.** Compound according to formula II, especially for use in the synthesis of compounds of formula I,

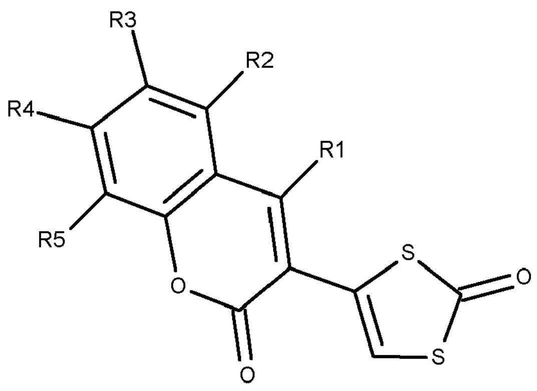

**II**

wherein R1-R5 have the same meaning as indicated in any one of claims 1 to 5.

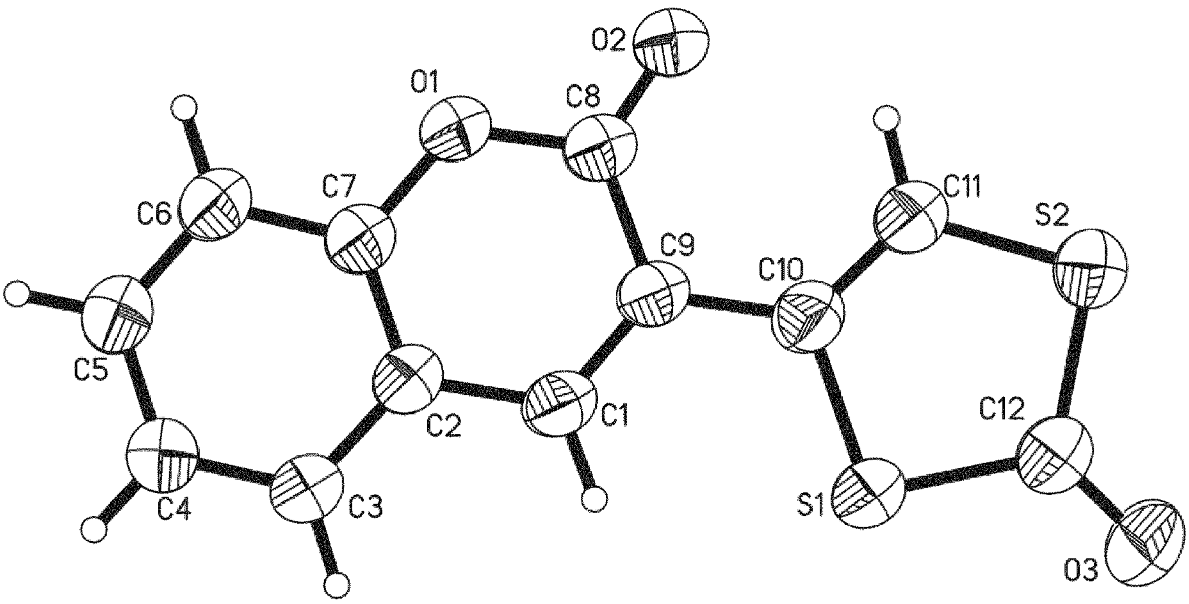

*Fig. 1a*

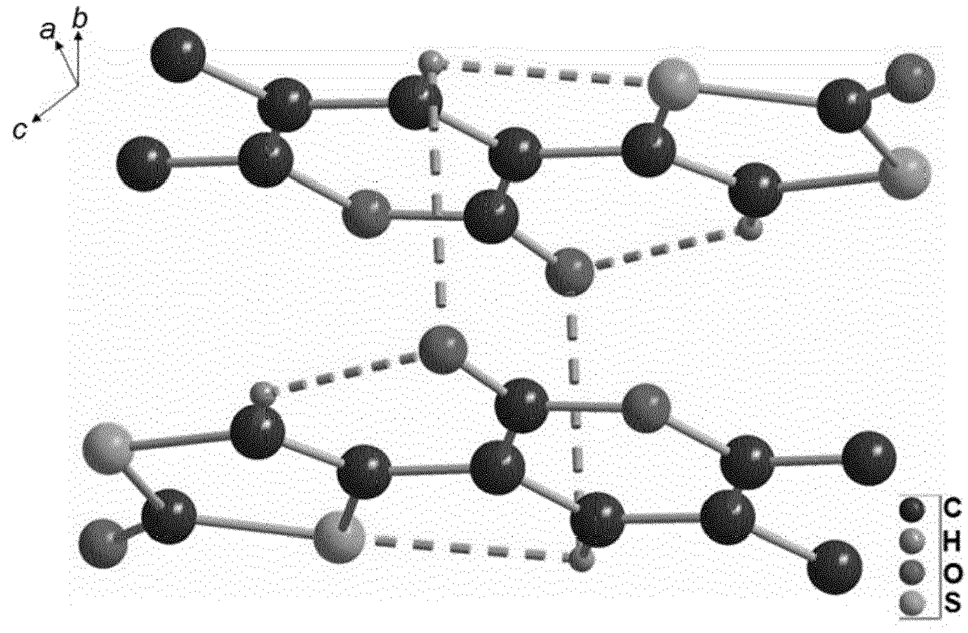


*Fig. 1b*

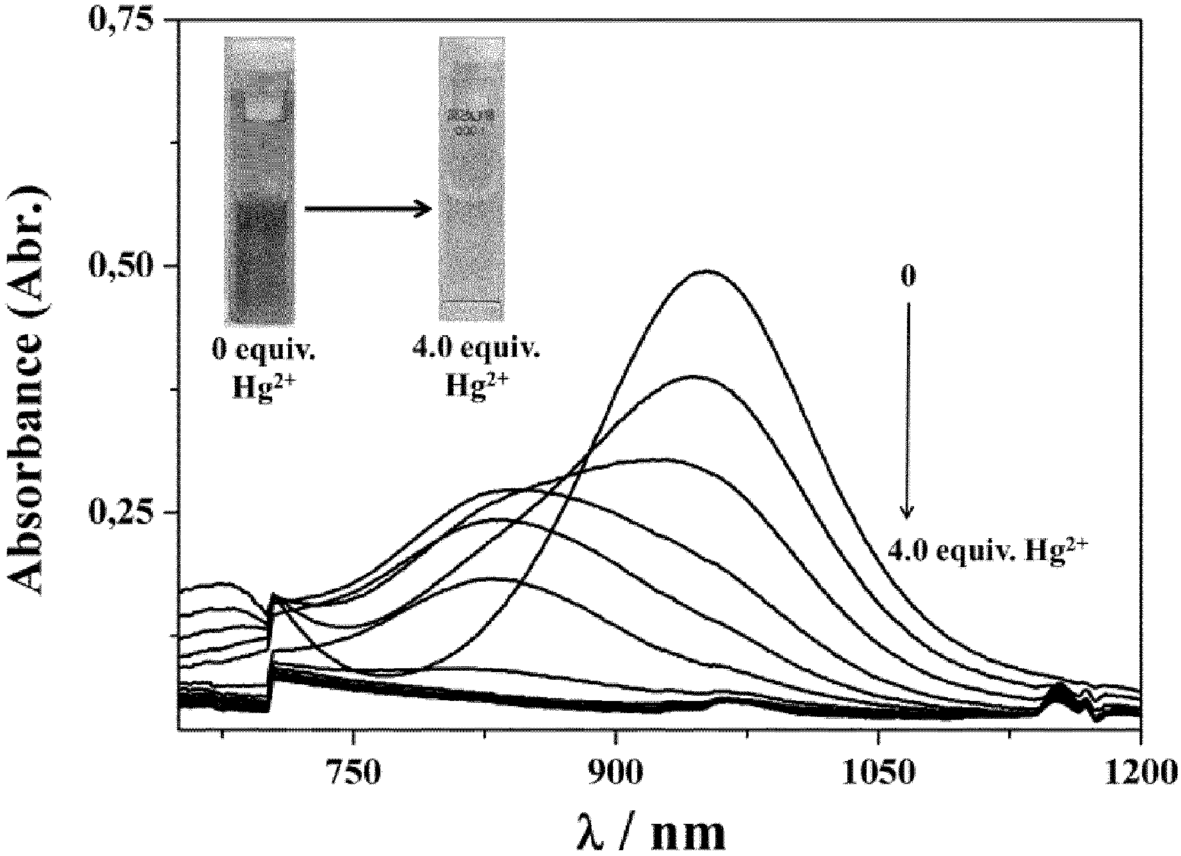
0,75
0,50
0,25
Absorbance (Abr.)
0 equiv.
Hg2+
4.0 equiv.
Hg2+
0
4.0 equiv. Hg2+
750
900
1050
1200
λ / nm

*Fig. 2*

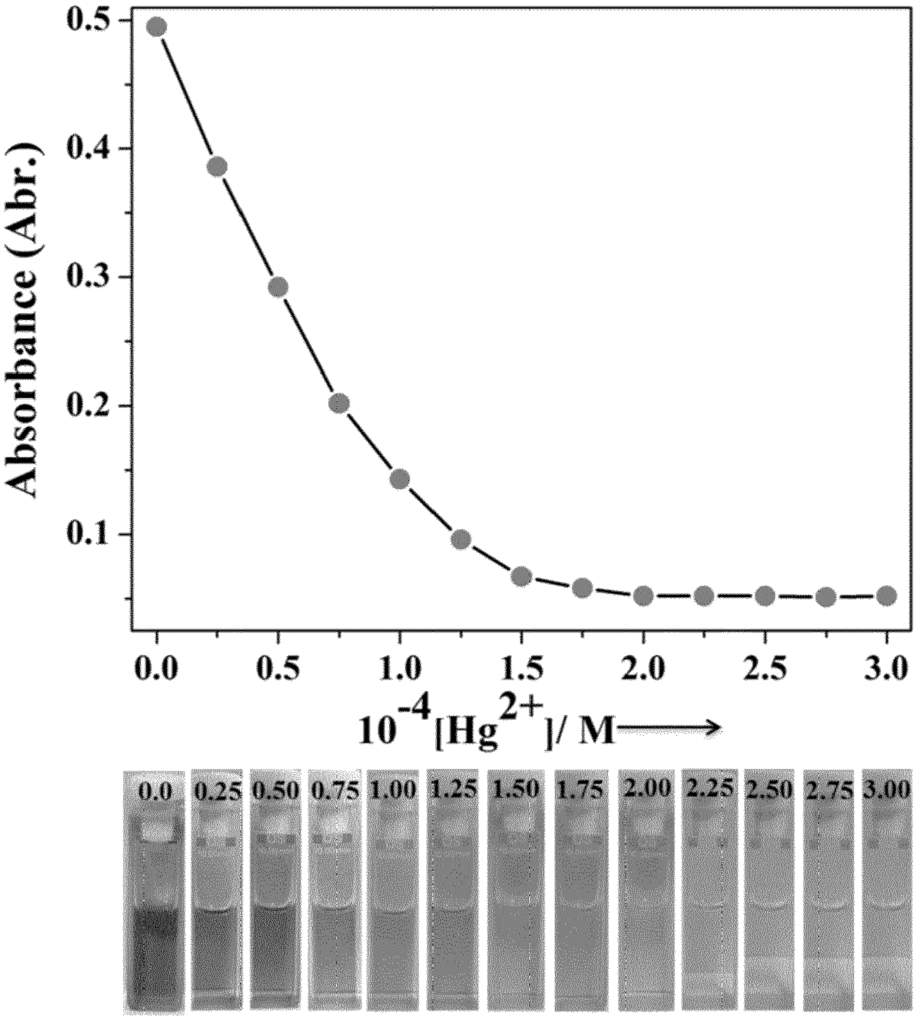
Absorbance (Abr.)
0.5
0.4
0.3
0.2
0.1
0.0
0.5
1.0
1.5
2.0
2.5
3.0
$10^{-4}[Hg^{2+}]$/ M
0.0 0.25 0.50 0.75 1.00 1.25 1.50 1.75 2.00 2.25 2.50 2.75 3.00

*Fig. 3*

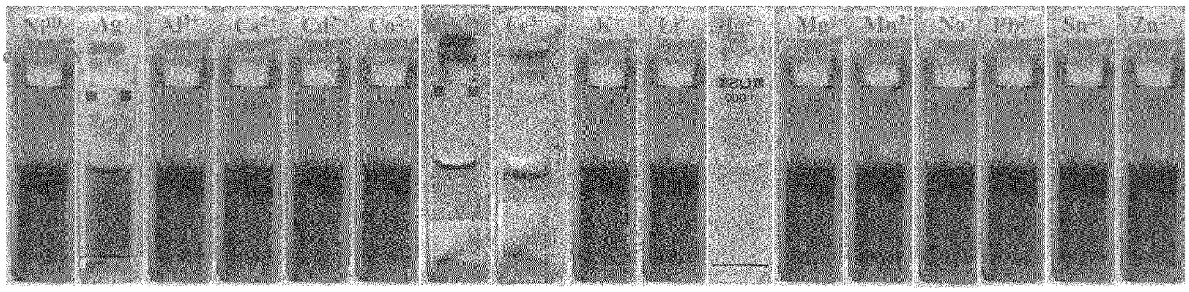

*Fig. 4*

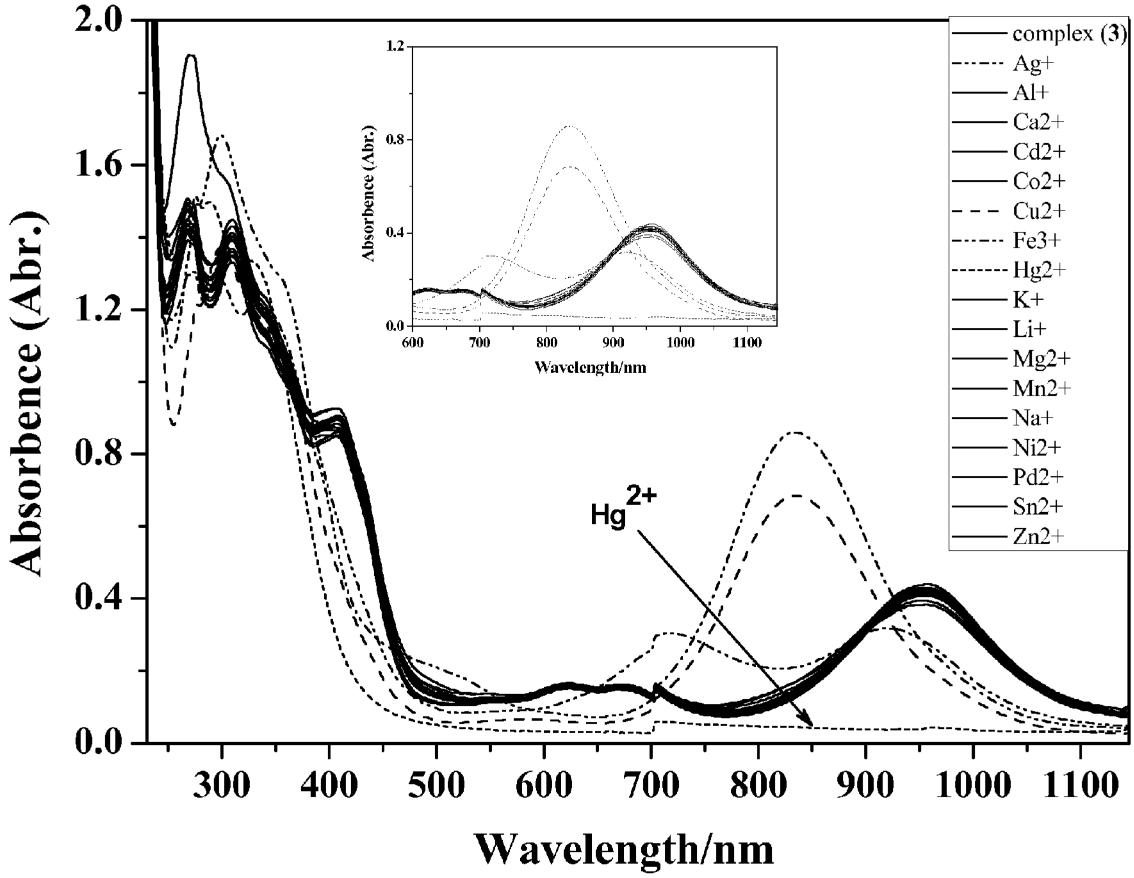
Absorbence (Abr.)
Wavelength/nm
Hg2+
complex (3)
Ag+
Al+
Ca2+
Cd2+
Co2+
Cu2+
Fe3+
Hg2+
K+
Li+
Mg2+
Mn2+
Na+
Ni2+
Pd2+
Sn2+
Zn2+

*Fig. 5*

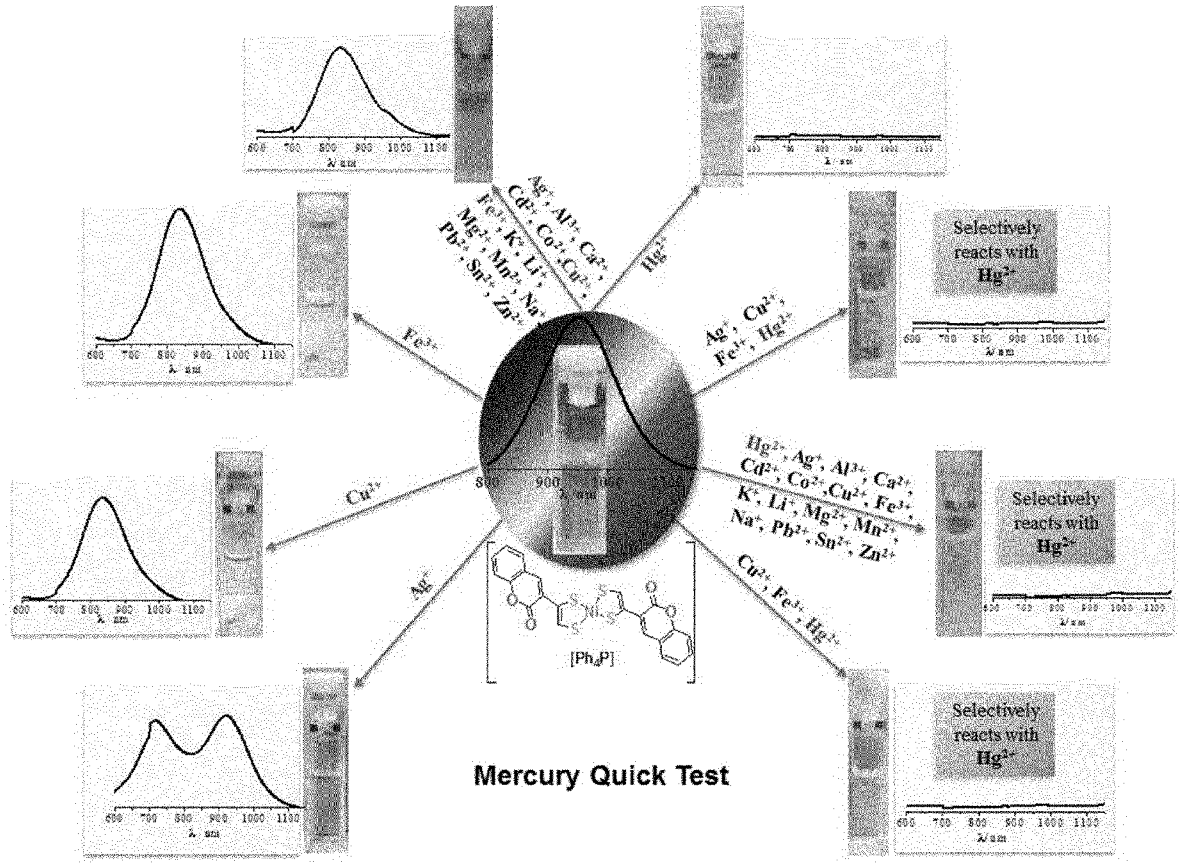

Ag+, Al3+, Ca2+, Cd2+, Co2+, Cu2+, Fe3+, K+, Li+, Mg2+, Mn2+, Na+, Pb2+, Sn2+, Zn2+
Hg2+
Fe3+
Ag+, Cu2+, Fe3+, Hg2+
Cu2+
Hg2+, Ag+, Al3+, Ca2+, Cd2+, Co2+, Cu2+, Fe3+, K+, Li+, Mg2+, Mn2+, Na+, Pb2+, Sn2+, Zn2+
Ag+
Cu2+, Fe3+, Hg2+
Selectively reacts with Hg2+
Selectively reacts with Hg2+
Selectively reacts with Hg2+
[Ph4P]
λ/nm
Mercury Quick Test


*Fig. 6*

Europäisches Patentamt
European Patent Office
Office européen des brevets

# EUROPEAN SEARCH REPORT

Application Number
EP 15 16 3318

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Ashta Chandra Ghosh: "Research - Highly selective visible and near-IR marking of Ni2+, Ag+ and Hg+ based on metal bis (dithiolene) complexes", , 1 January 2014 (2014-01-01), pages 1-2, XP055209651, Retrieved from the Internet: URL:http://astaghoshphd.blogspot.nl/p/research.html [retrieved on 2015-08-26] * page 1, last paragraph - page 2, paragraph 1 * | 1-14 | INV. C07D311/12 C07F9/54 C07D409/04 G01N33/00 G01N33/18 |
| X | -& Ashta Chandra Ghosh: "Highly selective visible and near-IR marking of Ni2+, Ag+ and Hg+ based on metal bis (dithiolene) complexes - ENLARGED", http://astaghoshphd.blogspot.nl/p/research.html, 1 January 2014 (2014-01-01), XP055209661, Retrieved from the Internet: URL:http://2.bp.blogspot.com/-L5M98VDEUy0/U4jZrEBNGfI/AAAAAAAAAUY/TT6i6NHY9I4/s1600/Untitled-1.tif [retrieved on 2015-08-26] * the whole document * ----- | 1-14 | |
| | | | TECHNICAL FIELDS SEARCHED (IPC) |
| | | | C07D C07F G01N |

1 The present search report has been drawn up for all claims

| Place of search | Date of completion of the search | Examiner |
|---|---|---|
| The Hague | 4 September 2015 | Eberhard, Michael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **E. M. NOLAN ; S. J. LIPPARD.** *Chem. Rev.,* 2008, vol. 108, 3443-3480
- **H. H. HARRIS ; I. J. PICKERING ; G. N. GEORGE.** *Science,* 2003, vol. 301, 1203
- **M. SALEEM ; R. ABDULLAH ; A. ALI ; B. J. PARK ; E. H. CHOI ; I. S. HONG ; K. H. LEE.** *Anal. Methods.,* 2014, vol. 6, 3588-3597
- **A. RENZONI ; F. ZINO ; E. FRANCHI.** *Environ. Res.,* 1998, vol. 77, 68-72
- **S. HARDY ; P. JONES.** *J. Chromatogr. A,* 1997, vol. 791, 333-338
- **P. B. TCHOUNWOU ; W. K. AYENSU ; N. NINASHVILI ; D. SUTTON.** *Environ. Toxicol.,* 2003, vol. 18, 149-175
- **J. GUTKNECHT.** *J. Membr. Biol.,* 1981, vol. 61, 61-66
- **M. HARADA.** *Crit. Rev. Toxicol.,* 1995, vol. 25, 1-24
- **N. LU ; C. SHAO ; Z. DENG.** *Analyst,* 2009, vol. 134, 1822-1825
- **Y.-K. YANG ; K.-J. YOOK ; J. TAE.** *J. Am. Chem. Soc.,* 2005, vol. 127, 16760-16761
- **X. ZHANG ; Y. XIAO ; X. QIAN.** *Angew. Chem. Int. Ed.,* 2008, vol. 47, 8025-8029
- **B.-C. YE ; B.-C. YIN.** *Angew. Chem. Int. Ed.,* 2008, vol. 47, 8386-8389
- **S. YOON ; E. W. MILLER ; Q. HE ; P. H. DO ; C. J. CHANG.** *Angew. Chem. Int. Ed.,* 2007, vol. 46, 6658-6661
- **J.-S. LEE ; M. S. HAN ; C. A. MIRKIN.** *Angew. Chem.,* 2007, vol. 119, 4171-4174
- **H. N. KIM ; W. X. REN ; J. S. KIM ; J. YOON.** *Chem. Soc. Rev.,* 2012, vol. 41, 3210-3244
- **A. P. DE SILVA ; H. Q. N. GUNARATNE ; T. GUNNLAUGSSON ; A. J. M. HUXLEY ; C. P. MCCOY ; J. T. RADEMACHER ; T. E. RICE.** *Chem. Rev.,* 1997, vol. 97, 1515-1566
- **B. ZHU ; W. WANG ; L. LIU ; H. JIANG ; B. DU ; Q. WEI.** *Sensor. Actuat. B-Chem,* 2014, vol. 191, 605-611
- **L. YUAN ; W. LIN ; K. ZHENG ; L. HE ; W. HUANG.** *Chemical Society Reviews,* 2013, vol. 42, 622-661
- **Z. DONG ; X. TIAN ; Y. CHEN ; J. HOU ; Y. GUO ; J. SUN ; J. MA.** *Dyes. Pigments,* 2013, vol. 97, 324-329
- **J. LIU ; D. WU ; C. DUAN ; Y. GUAN.** *Talanta,* 2013, vol. 105, 87-92
- **X. CHEN ; S.-W. NAM ; M. J. JOU ; Y. KIM ; S.-J. KIM ; S. PARK ; J. YOON.** *Org. Lett.,* 2008, vol. 10, 5235-5238
- **J. NI ; Q. LI ; B. LI ; L. ZHANG.** *Sensor. Actuat. B-Chem,* 2013, vol. 186, 278-285
- **Z. GUO ; W. ZHU ; M. ZHU ; X. WU ; H. TIAN.** *Chem. Eur. J.,* 2010, vol. 16, 14424-14432
- **M.-H. HA-THI ; M. PENHOAT ; V. MICHELET ; I. LERAY.** *Org. Lett.,* 2007, vol. 9, 1133-1136
- **L. PRAVEEN ; V. B. GANGA ; R. THIRUMALAI ; T. SREEJA ; M. L. P. REDDY ; R. L. VARMA.** *Inorg. Chem.,* 2007, vol. 46, 6277-6282
- **H. WANG ; Y. LI ; S. XU ; Y. LI ; C. ZHOU ; X. FEI ; L. SUN ; C. ZHANG ; Y. LI ; Q. YANG.** *Org. Biomol. Chem.,* 2011, vol. 9, 2850-2855
- **N. WANICHACHEVA ; S. WATPATHOMSUB ; V. S. LEE ; K. GRUDPAN.** *Molecules,* 2010, vol. 15, 1798-1810
- **L. MARBELLA ; B. SERLI-MITASEV ; P. BASU.** *Angew. Chem., Int. Ed.,* 2009, vol. 48, 3996-3998
- **A. C. GHOSH ; J. K. REINHARDT ; M. K. KINDERMANN ; C. SCHULZKE.** *Chem. Commun.,* 2014, vol. 50, 10102-10104
- **O. JEANNIN ; R. CLÉRAC ; M. FOURMIGUÉ.** *Journal of the American Chemical Society,* 2006, vol. 128, 14649-14656
- **T. ANJOS ; S. J. ROBERTS-BLEMING ; A. CHARLTON ; N. ROBERTSON ; A. R. MOUNT ; S. J. COLES ; M. B. HURSTHOUSE ; M. KALAJI ; P. J. MURPHY.** *Journal of Materials Chemistry,* 2008, vol. 18, 475-483
- **M. FOURMIGUE ; J. N. BERTRAN.** *Chemical Communications,* 2000, 2111-2112
- **B. S. LIM ; D. V. FOMITCHEV ; R. H. HOLM.** *Inorg. Chem.,* 2001, vol. 40, 4257-4262
- **V. MADHU ; S. K. DAS.** *Inorg. Chem.,* 2008, vol. 47, 5055-5070
- **L. N. DAWE ; J. MIGLIOI ; L. TURNBOW ; M. L. TALIAFERRO ; W. W. SHUM ; J. D. BAGNATO ; L. N. ZAKHAROV ; A. L. RHEINGOLD ; A. M. ARIF ; M. FOURMIGUÉ.** *Inorg. Chem.,* 2005, vol. 44, 7530-7539
- **D. F. EVANS.** *J. Chem. Soc.,* 1959, 2003-2005
- **D. F. EVANS ; G. V. FAZAKERLEY ; R. F. PHILLIPS.** *J. Chem. Soc. (A),* 1971, 1931-1934
- **D. F. EVANS ; D. A. JAKUBOVIC.** *J. Chem. Soc., Dalton Trans.,* 1988, 2927-2933

- **H. P. FRITZ ; K. E. SCHWARZHANS.** *J. Organomet. Chem.,* 1964, vol. 1, 208-211
- **D. F. EVANS ; T. A. JAMES.** *J. Chem. Soc., Dalton Trans.,* 1979, 723-726
- **Q. FANG ; Y.-M. SUN ; X.-Z. YOU.** *Chin. J. Chem. Phys.,* 1992, vol. 1, 129
- **M. SHORTREED ; R. KOPELMAN ; M. KUHN ; B. HOYLAND.** *Anal. Chem.,* 1996, vol. 68, 1414-1418
- **A. ALTOMARE ; G. CASCARANO ; C. GIACOVAZZO ; A. GUALARADI.** *J. Appl. Crystallogr.,* 1993, vol. 26, 343
- **G. M. SHELDRICK.** *Acta Crystallogr. A,* 2008, vol. 64, 112-122
- **L. FARRUGIA.** *J. Appl. Crystallogr.,* 1999, vol. 32, 837-838
- **E. A. BOUDREAUX ; D. J. MILLER.** *Inorg. Nucl. Chem. Lett.,* 1966, vol. 2, 59-61